# EUROPEAN PATENT APPLICATION

(11) **EP 3 499 222 A1**
(43) Date of publication of application: **19.06.2019**
(21) Application number: 18210967.8
(22) Date of filing: 07.12.2018
(51) Int. Cl.: G01N 21/89, G01N 33/36

(54) **OPTICAL YARN SENSOR AND METHODS OF CONTROLLING IT**

(30) Priority: 12.12.2017 CZ 20170796
(71) Applicant: MASCHINENFABRIK RIETER AG, 8406 Winterthur (CH)
(72) Inventor: Kousalik, Pavel, 562 03 Usti nad Orlici, Hylvaty (CZ); Beran, Zdenek, 563 01 Lanskroun, Ostrovske Predmesti (CZ)
(74) Representative: Musil, Dobroslav

(57) **Abstract**

The invention relates to an optical yarn (3) sensor which comprises a radiation source (1) which is through an optical element (12) directed to a detection zone (2) for the passage of yarn (3), and at least one sensing element (4) of the yarn (3) parameters is directed to this zone (2), whereby the radiation source (1) comprises at least two radiating elements (11) and is connected to a power supply and control block (5), and the sensing element (4) is connected to a control and evaluation block (6), wherein the power supply and control block (5) and the control and evaluation block (6) are coupled to a control block (7) of the optical yarn (3) sensor. At least two of the radiating elements (11) emit the same radiation and the optical element (12) is formed by an optical element for collimation or slight divergence of the light beams from all the individual radiating elements (11) with the same radiation to one part of the detection zone (2), whereby the control block (7) is provided with means for both parallel and sequential control of each radiating element (11) with the same radiation.

## Description

### Technical field

The invention relates to an optical yarn sensor which comprises a radiation source which is directed through an optical element to a detection zone for the passage of yarn and at least one sensing element of yarn parameters which is directed to this zone, whereby the radiation source comprises at least two radiating elements and is connected to a power supply and control block and the radiation sensor is coupled to a control and evaluation block, the power supply and control block and the control and evaluation block being coupled to a control block of the optical yarn sensor.

In addition, the invention relates to methods of controlling a radiation source of an optical yarn sensor, wherein the operation of at least two radiating elements is controlled.

### Background art

WO 2011 026 249 discloses a device which comprises two radiation sources placed in different parts of a common housing which emit radiation of different spectral characteristics. The two sources of radiation emit radiation beams such that they overlap in their central part, and through this zone, which is the detection zone of a sensor, passes the yarn scanned during the measurement. The edge portions of each radiation beam are situated in different parts of the detection zone. The yarn sensor is directed into the area of the detection zone irradiated by the overlapping beams of the two radiation sources. The mutually overlapping portions of the two radiation beams of different spectral characteristics, i.e. different colours, ensure that the scanned yarn is irradiated and scanned by all relevant radiation components, which is advantageous particularly for detecting color defects in the yarn.

Similar is the solution according to WO 2007 010 325 in which there are also two sources of radiation, one being a source of visible radiation and the other a source of infrared radiation, wherein two reflected light detectors are assigned to the yarn path, one being a visible radiation detector and the other an infrared radiation detector. The detectors are connected to an evaluation device which evaluates the difference between the signals from the two detectors in order to detect the presence of a contaminant, namely by determining that the difference between the two detectors exceeded the predefined limit. To create a homogeneous radiation field along a specific length of yarn, i one of the two radiation sources in one embodiment is provided with a pair of infrared radiating elements and one radiating element in the visible region of light, whereas the other of the pair of radiation sources is provided with a pair of radiating elements in the visible region of light and one infrared radiating element. While the yarn is scanned, all the radiating elements of both sources of radiation emit light.

US 2006/0164646 (US 7 333 203) discloses an optical yarn sensor which comprises one light source in which are arranged elements emitting light in at least two different wavelengths in the visible range of the spectrum. For example, the light source is in the form of an RGB diode which is capable of independently controlling each of its light channels, that is, each of the light-emitting elements R - G - B, so that each of them can emit light independently of the others. The RGB diode has a common translucent housing which acts at the same time as an optical element, so that the individual RGB light-emitting elements basically emit light directed at least partially to the central axis of the housing. For spatial reasons, however, the RGB light fields do not fully overlap, but for the purpose of detecting colour defects in the yarn it is sufficient for the light beams of all the light-emitting elements to overlap in the central axis of the housing. This arrangement is advantageous for providing a compact device for scanning yarn color unevenness where it is necessary to illuminate the multicolored impurities by radiation of different wavelengths so that these impurities are detectable, typically by a detector of reflected radiation.

As is known, the radiating elements of light-emitting diodes, whether they are LEDs or laser diodes, are aging over time, or, in other words, they degrade in the course of their radiation, which means that after a certain period of time their light output or also other characteristics are no longer sufficient or a complete failure of one of the radiating elements will occur and that will lead to the failure of the entire light-emitting diode. Therefore, it is not a question of whether the light source of the light-emitting diode will have a fault, but when this fault will occur. The failure of the radiation source, even if it is only one of its radiating elements, destroys the basic function of the optical yarn sensor which must be replaced to renew the functionality of the optical yarn sensor, which requires time, human labor and the shutdown of the machine on which the respective service operation is performed. Theoretically, it is possible to replace only the light source in the body of the optical yarn sensor. This, however rather demanding and inefficient due to the current single-purpose and compact designs.

The aim of the invention is to eliminate or at least reduce the disadvantages of the background art resulting from faults of light-emitting diodes or even only one of their radiating elements, especially to extend the lifetime of the optical yarn sensor as a whole without a servicing intervention and possibly to increase the versatility of the yarn quality sensor in terms of sensing geometric parameters or color characteristics of the yarn.

### Principle of the invention

The aim of the invention is achieved by an optical yarn sensor whose principle consists in that among its radiating elements there are at least two with the same radiation emitted, and the optical element is formed by an optical element for collimation or slight divergence of the radiation beams from all the individual radiating elements with the same radiation in one part of the detection zone, wherein the control block is provided with means for parallel and sequential control of each of the radiating elements with the same radiation.

The invention also relates to several methods of controlling the optical sensor, in which the operation of at least two radiating elements with the same radiation is controlled in various relationships with the operation of a workstation or the operating conditions at a workstation or with the yarn parameters to be measured.

In principle, the invention employs the multiplication of the same radiating elements in the radiation source to increase the lifetime and reliability of the radiation source using simultaneous or even alternating or otherwise interdependent lighting of the individual radiating elements, including multicolored radiating elements. Another advantage is that such a radiation source can be created as one undismountable unit, whose price is low and, at the same time, its replacement in case of need is quick and efficient. In addition, the invention enables to effectively eliminate possible decrease in luminosity of one of the radiating elements by means of another radiating element, by increasing the current to the radiating element, etc., which enables further optimization of the light source for the specific conditions at the workstation.

### Description of the drawings

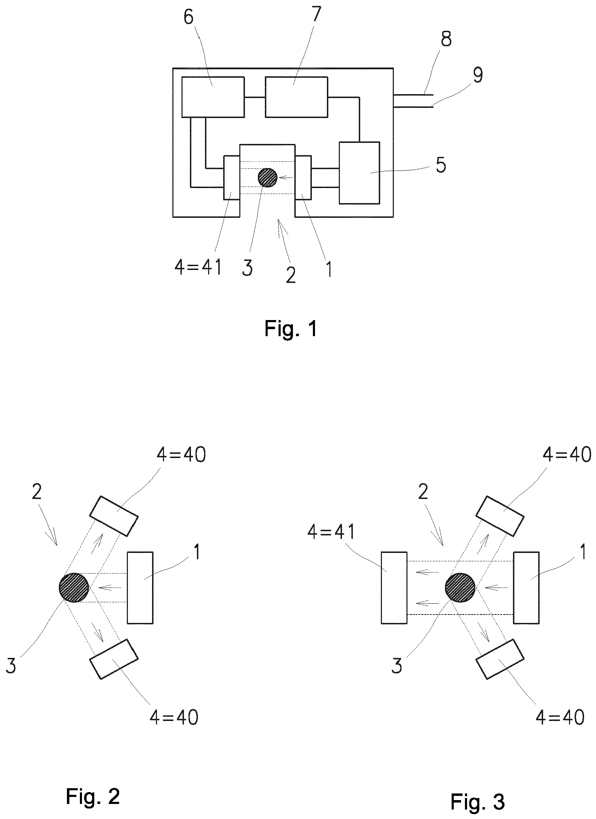

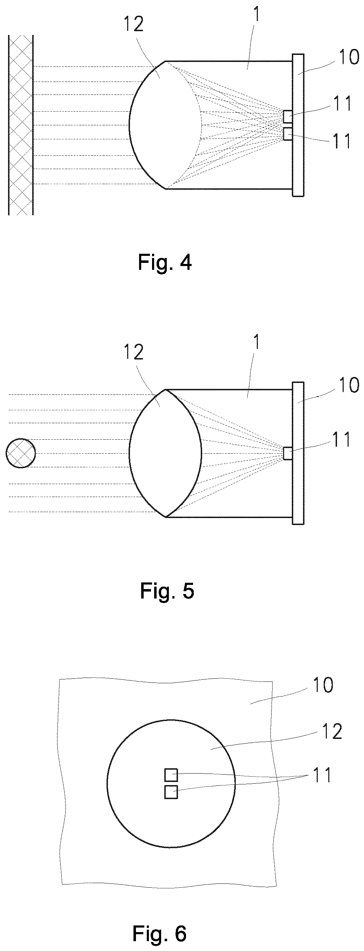

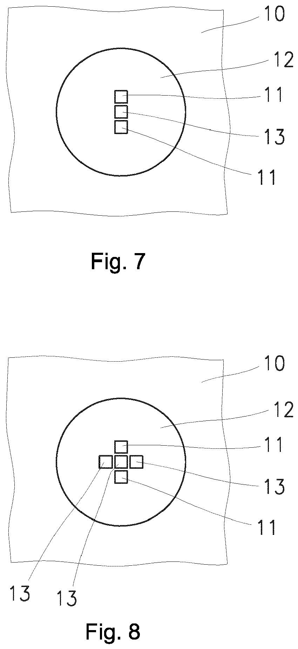

The invention is schematically represented in the drawing, where Fig. 1 shows an embodiment of an optical yarn sensor, wherein a detection zone for the passage of yarn is arranged between a sensing element and a radiation source, Fig. 2 shows an embodiment of an optical yarn sensor for sensing radiation reflected from the yarn, Fig. 3 shows a combination of embodiments of Fig. 1 and Fig. 2, Fig. 4 is a side view of an exemplary embodiment of the radiation source according to the invention, Fig. 5 is an axial view of an exemplary embodiment of the radiation source according to the invention, Fig. 6 is a front view of a support plate with a pair of radiating elements emitting the same radiation, Fig. 7 is a front view of the support plate with a pair of radiating elements emitting the same radiation and with at least one further radiating element assigned to them and finally, Fig. 8 is a front view of the support plate with a pair of radiating elements emitting the same radiation and three further radiating elements.

### Examples of embodiment

The invention will be described with reference to several examples of embodiment of an optical yarn sensor with a radiation source and with reference to several examples of embodiment of the radiation source for the optical yarn sensor.

The optical yarn **3** sensor comprises a radiation source **1,** which is directed to a detection zone **2,** through which the yarn **3** passes during measurement. At least one sensing element **4** is further assigned to the detection zone **2.** The sensing element **4** is formed by a suitable means with at least one element sensitive to radiation, ideally by at least one row of elements sensitive to radiation (a CCD element or a CMOS element).

The radiation source **1** is connected to a power supply and control block **5**. The sensor **4** of radiation is connected to a control and evaluation block **6.** The power supply and control block **5** and the control and evaluation block **6** are coupled to the control block **7** of the optical yarn **3** sensor. The optical yarn **3** sensor is coupled through a communication **bus 8** to the unillustrated superior control block, e.g. the control block of a workstation of a yarn manufacturing textile machine, or to the unillustrated control block of a group of workstations or to the unillustrated control block of the entire machine. The optical yarn **3** sensor is also connected to a power supply **9.**

Figs. 1 to 3 show the basic concepts of an arrangement of an optical yarn **3** sensor used. Fig. 1 shows a concept of an arrangement of the optical yarn **3** sensor in which yarn **3** parameters are determined in the detection zone **2** according to the extent of the shading of the sensing element **4=41** by the yarn **3.** Fig. 2 shows a concept od an arrangement of an optical yarn **3** sensor in which the yarn **3** parameters in the detection zone **2** are determined by means of detecting the reflected radiation, i.e. the radiation emitted by the radiation source **1** to the detection zone **2** and reflected from the yarn **3** and detected by the sensing element **4=40** of the reflected radiation. Fig. 3 shows a concept of a combined arrangement of the optical yarn **3** sensor of the embodiment according to Figs. 1 and 2, in which the yarn **3** parameters in the detection zone **2** are determined either according to the extent of the shading of the sensing element **4=41** by the yarn **3** and they are determined by detecting the radiation reflected, i.e. the radiation emitted by the radiation source **1** to the detection zone **2** and reflected from the yarn **3** and detected by the sensing element **4=40** of the reflected radiation. In the embodiments of Figs. 2 and 3, the control block **5,** the control and evaluation block **6,** the control block **7,** the communication **bus 8** and the power supply **9** are not shown, but it is apparent that these elements **5** to **9** are adequately represented here.

Figures 4 and 5 show a basic arrangement of the radiation source **1** which comprises a base plate **10** on which are mounted at least two radiating elements **11** with the same radiation emitted, i.e., e.g., with the same wavelength of the emitted radiation, or with the same color of the emitted radiation or with the same multicolored radiation, etc. It is essentially about placing at least two "identical" radiating elements **11** formed ideally by the same LED chips or the same laser diode chips on a common support plate **10** immediately next to each other, ideally as close to each other as possible, as can be seen in Figure 6. An optical element **12** for collimation or slight divergence of radiation beams from all the individual radiating elements **11** is arranged In the direction of radiation, in front of the radiating elements **11** and the detection zone **2** of the optical yarn **3** sensor. In the exemplary embodiment, the base plate **10** with the radiating elements **11** and the optical element is integrated into one undismountable source **1** of radiation. This solution considerably improves radiation quality and reliability of the source **1** because collimation of radiation is not affected by tolerances and inaccuracies during the installation and operation of the optical sensor **3.**

In the exemplary embodiment shown in Fig. 7, at least two radiating elements **11** with the same radiation emitted are mounted on the base plate **10**, i.e., e.g., with the same wavelength of the emitted radiation, or with the same color of the emitted radiation or with the same multicolored emitted radiation, etc., whereby to the radiating elements **11** with the same radiation emitted is assigned at least one additional radiating element **13** with, i.e. with the emitted radiation wavelength different from the radiating elements **11**, or with different color of the emitted radiation or with different multicolored emitted radiation, etc. In principle, two "identical" radiating elements **11** formed ideally by the same LED chips or the same laser diode chips and at least one additional radiating element **13**, whereby also the additional radiating element **1**3, or the additional radiating elements **13**, is/are located on the common plate **10** ideally as close as possible to each other and to the radiating elements **11**.

In the exemplary embodiment shown in Fig. 8, two radiating elements **11** with the same emitted radiation are mounted on the base plate **10**, whereby to the elements **11** with the same emitted radiation are assigned three additional radiating elements **13** emitting radiation different from that of the radiating elements **11**, i.e. with different wavelength of the emitted radiation, or with different color of the emitted radiation or with different multicolored emitted radiation, etc.

In an embodiment not shown, mounted on the base plate **10** is at least another radiating element with radiation different both from the radiating elements **11**, and from the additional radiating element **13**, or additional radiating elements **13**, i.e. with a different wavelength of the emitted radiation or with different color of the emitted radiation or with different multicolored emitted radiation, etc.

In the direction of the radiation, in front of the radiating elements **11** and at least one possible additional radiating element **13**, i.e. between the radiating elements **11**, **13** and the detection zone **2** the optical yarn **3** sensor, is arranged an optical member **12** for collimation or slight divergence of the light beams from all the individual radiating elements **11**, **13** to one area of the detection zone **2**. Under the term "slight divergence" of the light beams we understand divergence of the light beams from all the individual radiating elements **11**, **13** relative to the detection yone **2** up to ±2,5°.

By means of such arrangement of the system of radiating elements **11**, **13**, etc., at least two of which emit the same radiation, e.g., with the same wavelength of the emitted radiation, or with the same color of the emitted radiation or with the same multicolored emitted radiation, etc., a complex and versatile source of radiation is created for a variety of yarn scanning applications, while achieving a high durability of the radiation source **1** thanks to the use of at least two identical radiating elements.

In an exemplary embodiment, the radiation source **1** comprises a pair of LEDs emitting the same visible light, which constitute the radiating elements **11** for measuring the yarn **3** dimensional characteristics, since this use of the optical yarn **3** sensor is the most common. In this example of embodiment, the radiation source **1** further comprises at least two additional radiating elements **13**, which have mutually different radiation emitted and at the same time their radiation emitted differs from the radiation emitted by the radiating elements **11.** In this example of embodiment, the radiating elements **11** selected are radiating elements with the lowest lifetime or reliability.

The device according to the invention can be used due to its arrangement in many different ways, the primary purpose of the invention being to increase the service life and reliability of the radiation source **1** while maintaining the intensity of the emitted radiation. The invention primarily uses the fact that individual radiating elements **11**, **13** are designed to emit the required radiation intensity separately.

In order to increase the service life and reliability of the radiation source **1** according to the first method, the control block **7** is provided with means for periodical sequential switching of the same radiating elements **11**, or possibly **13**, so that in the detection zone **2** there is the required intensity of radiation, with only one of the same radiating elements **11** or possibly **13** emitting light, or at first one of the same radiating elements **11**, or possibly **13**, operates for the whole period of its service life and after the end of its service life or the decrease in its luminous intensity below the set limit, another of the same radiating elements **11**, or possibly **13**, is activated and continues to operate in place of the previous "used up" same radiating element **11,** or possibly **13.**

To increase the service life and reliability of the radiation source **1** according to the second method, the control block **7** is provided with means for feeding simultaneously to at least two identical radiating elements **11,** or possibly **13**, a lower current than the supply current to achieve a nominal luminous power such that the desired radiation intensity is reached in the detection zone **2** in the sum of the current lower luminous output of each of the operating radiating elements **11**,**13.** This means, therefore, that at least two of the same radiating elements **11**, or possibly **13**, are operating simultaneously, whereby each of these identical radiating elements **11**, **13** emits less radiation, the sum of the intensity of these radiations in the detection zone corresponding to the required luminous intensity in the detection zone **2**. By this limited power supply to each of the simultaneously operating identical radiating elements **11**, **13**, the service life of the radiation source **1** is extended, and this lifetime extension of the used radiating elements **11**,**13** is due to the logarithmic dependence of the lifetime of the radiating elements **11**,**13** on the size of the supply current even greater than a simple sum of the lifetime of the individual radiating elements **11, 13** used.

To achieve further increase in the service life of the radiation source **1**, the control block **7** is provided with means for controlling the current flowing through the radiating elements **11**, **13**. During the operation, the supply power is adjusted in such a manner that the desired intensity of radiation is achieved in the detection zone **2**. At times when the signals from the sensing elements **4 (41**, **40)** are unaffected by the yarn (e.g., when a yarn break occurs), the signal levels from the sensing elements **4 (41**, **40)** are measured and if, for example, there is a decrease caused by the decrease in the intensity of the radiation emitted due to the aging of the radiating element **11**, **13,** the control block increases the current supply to the radiating element so that the desired radiation intensity in the detection zone is reached again. The above-described complex radiation source **1** comprising multicolor radiating elements **11**, **13**, etc., enables a variety of yarn monitoring applications to run, without the need for all conceivable applications to be recognized and without the need to install an optical yarn **3** sensor at the workstation with desired properties and desired variability wavelengths of radiation. In addition to long life and reliability when the radiating elements **11**, **13** are operating similtaneously etc., such a complex radiation source **1** for the fiber optic sensor allows, e.g., automatic switching of the active radiating elements **11**, **13,** e.g., based on the signal from the sensing elements **4** (**41**, **40**) for the measurement of the diameter and/or the reflected light. Furthermore, according to one application, the radiation of the required wavelength is used for monitoring a specific yarn parameter, e.g., the red color is used for the clearer when detection of the foreign fibers is switched off, and the green color is used when the detection of foreign fibers is switched on, e.g., for example, individual colors are switched according to the color of the processed fibers, or according to the color of the yarn, to ensure detection of foreign fibers for the entire color spectrum of the yarns, etc. A radiating element **11**, **13** etc. with red light is used for shades of red yarn, a radiating element **11**, **13** etc. with green light is used for green yarns, a radiating element **11**, **13** etc. with blue light is used for blue yarns.

In addition, it is possible to realize the switching of the radiating elements **11**, **13**, etc., by pulse width modulation (PWM) so that the radiating element **11, 13**, etc., of each color is switched at a different time and hence there is no interaction of yarn **3** measurement in those applications, where it is inappropriate, for example in the detection of foreign fibers in the yarn **3,** only the radiating element **11**, **13** etc. with the green light is switched and at that time the diameter of the yarn **3** is not measured, whereupon follows switching to the radiating element **11**, **13** etc. with the red light, in which the yarn **3** diameter is measured . At the same time, however, it is possible to measure also colored impuriies in this red light. It is advantageous to use this control even in cases when, for example, the sensivity of the sensing elements **4** (**41**, **40**) depends on the received wavelength. For example, if the sensing element **41** is the most sensitive to red color, and sensing element **40** is sensitive to green, then the sensing element **41** measures only when the red radiating element is lit and sensor element **42** measures at the time when the green radiating element is lit. Furthermore, it is possible to realize the switching of the radiating elements **11**, **13**, etc., sequentially (e.g., R, G, B, R, G, B, etc.) so that for each scanned yarn **3** sample thus scanned under the sequentially colored light, information about the reflection of the radiation is obtained for different colors of the radiation source **1** and by subsequent processing of this information it is possible to determine the color of the scanned yarn **3** and at the same time even the color of the impurities in the yarn **3**, which is advantageous, for example, for further processing of the yarn **3**, when some colors of the impurities, for example of foreign fibers, are not necessarily inappropriate because the scanned yarn will, for example, be dyed in the next step to the same color as that of the yarn **3** or the foreign fiber in the yarn **3** or, on the contrary, some colors of the impurities or foreign fibers may be less acceptable than other colors, etc.

The solution according to the invention can also be operated in the mode of concurrent radiation of at least two radiating elements **11**, **13**, etc. with different wavelengths of radiation or in the mode of uninterrupted radiation of one of the radiating elements **11**, **13**, etc., and short-term switching of the other of the radiating elements **11,** with a different wavelength of radiation, or in the mode of the repeated gradual switching of the individual radiating elements **11, 13,** etc. according to the color of the processed fibers, or according to the color of the yarn produced in order to ensure detection of foreign fibers in the yarn **3** for the full color spectrum of the yarn, wherein, e.g., a radiating element **11**, **13** etc. with red light is used for shades of red yarn, a radiating element **11**, **13** etc. with green light is used for green yarns, a radiating element **11**, **13** etc. with blue light is used for blue yarns, radiating elements **11**, **13** etc. with green and red light are used for yellow yarns, the radiating elements **11**, **13** etc. with blue and red light are used for purple yarns, radiating elements **11**, **13** etc. of all channels of RGB are used for white yarns, etc.

In order to achieve optimal results, the sensitivity of the sensing element **4** according to a preferred embodiment depends on the color of the radiation of the currently active radiating element **11**, **13** etc. or of several radiating elements **11**, **13.**

## Claims

1. An optical sensor of yarn (3) comprising a radiation source (1), which is through an optical element (12) directed to a detection zone (2) for the passage of yarn (3), and at least one sensing element (4) of yarn (3) parameters which is further directed to this zone (2), whereby the radiation source (1) comprises at least two radiating elements (11) and is connected to a power supply and control block (5), the sensing element (4) is connected to a control and evaluation block (6), wherein the power supply and control block (5) and the control and evaluation block (6) are coupled to a control block (7) of the optical sensor of yarn (3), **characterized in that** at least two of the radiating elements (11) have the same radiation and the optical element (12) is formed by an optical element for collimation or slight divergence of the light beams from all the individual radiating elements (11) with the same radiation to one part of the detection zone (2), whereby the control block (7) is provided with means for both parallel and sequential control of each of the radiating elements (11) with the same radiation.

2. The optical sensor of yarn according to claim 1, **characterized in that** to the radiating elements (11) with the same radiation is assigned at least one additional radiating element (13) with radiation different from the radiating elements (11) with the same radiation, whereby the control block (7) is provided with means for both parallel and sequential control of each of the radiating elements (11, 13).

3. The optical sensor of yarn according to claim 2, **characterized in that** to the radiating elements (11) with the same radiation and to the additional radiating element (13) is assigned at least one additional radiating element with different radiation from both the radiating elements (11) with the same radiation and the additional radiating element (13).

4. The optical sensor of yarn according to any of claims 1 to 3, **characterized in that** the radiating elements (11, 13) are located immediately next to each other on a common support plate (10).

5. The optical sensor of yarn according to claim 4, **characterized in that** the common support plate (10) with the radiating elements (11, 13) and the optical element (12) are integrated into one undismountable complex.

6. A method of controlling the radiation source of the optical sensor of yarn according to any of claims 1 to 5, in which the operation of at least two radiating elements (11) with the same radiation is controlled, **characterized in that** each of at least two of the same radiating elements (11) with the same radiation is switched periodically and repeatedly, so that there is the required intensity of the radiation in the detection zone (2), while always only one of the same radiating elements (11) with the same radiation emits radiation.

7. The method of controlling the radiation source of the optical sensor of yarn according to any of claims 1 to 5, in which the operation of at least two radiating elements (11) with the same radiation is controlled, **characterized in that** at first one of the radiating elements (11) with the same radiation is switched for the whole period of its service life and after the end of its service life or after decrease in its radiation intensity below the set limit, another of the radiating elements (11) with the same radiation is activated.

8. The method of controlling the radiation source of the optical sensor of yarn according to any of claims 1 to 5, in which the operation of at least two radiating elements (11) with the same radiation is controlled, **characterized in that** at least two of the radiating elements (11) with the same radiation are switched simultaneously, whereby each of these elements (11) is powered by a lower current in comparison with the supply current for nominal light output such that, in the sum of the lower light outputs of the operating radiating elements (11) with the same radiation, the required radiation intensity is reached in the detection zone (2) .

9. The method of controlling the radiation source of the optical sensor of yarn according to any of claims 1 to 5, in which the control block (7) is provided with means for controlling the intensity of radiation in the detection zone (2) by means of the current flowing through the radiating elements (11, 13), **characterized in that** the required intensity of radiation in the detection zone (2) is maintained constant, whereby a change in the intensity of radiation in the detection zone (2) is sensed by the sensing elements (4) and according to the signals from the sensing elements (4) the intensity of radiation in the detection zone (2) is corrected to the required value by changing the light output of the radiating elements (11, 13).

10. The method according to any of claims 6 to 9, in which the operation of at least two radiating elements (11) is controlled, **characterized in that** each of at least two radiating elements (11, 13) with the same radiation is switched sequentially and/or in parallel so that the required intensity and/or color of radiation in the detection zone (2) is achieved.
